# EUROPEAN PATENT APPLICATION

(11) **EP 4 458 492 A1**
(43) Date of publication of application: **06.11.2024**
(21) Application number: 24171739.6
(22) Date of filing: 22.04.2024
(51) Int. Cl.: B22F 7/00, A61F 2/30, B22F 10/28, B22F 10/40, B22F 10/47, B22F 10/62, B22F 10/66, B33Y 10/00, B33Y 40/20, B33Y 80/00

(54) **BREAKAWAY COVERS FOR USE IN PROCESSING BUILD STRUCTURES WITH POROUS REGIONS**

(30) Priority: 24.04.2023 US 202363461357 P
(71) Applicant: Howmedica Osteonics Corporation, Mahway, New Jersey 07430 (US)
(72) Inventor: PARKER, Brad A., Warsaw, Indiana 46582 (US); WALSH, Gearoid, Ennis, Co. Clare V95 X8P2 (IE); BERNSTEIN, Henry, Teaneck, New Jersey 07666 (US); BOBISH, Christopher S., Middletown, New York 10940 (US); SPLIETH, Roy Philip, Central Valley, New York 10917 (US)
(74) Representative: Eisenführ Speiser

(57) **Abstract**

An in-process build structure includes a first build structure portion, a second build structure portion, and a cover. The second build structure portion is attached to the first build structure portion and defined by a plurality of porous portion struts. The cover at least partially overlies the second build structure portion and is attached to a remainder of the build structure by breakaway struts having breakaway strut diameters less than porous portion strut diameters of the porous portion struts. The in-process build structure is subjected to a surface treatment while the cover is attached to a remainder of the in-process build structure. The cover is removed from the remainder of the in-process build structure after the surface treatment.

## Description

### FIELD

The present invention generally relates to additive manufacturing (AM), and in particular relates to structures and systems for improving the efficiency in the surface treatment of articles following initial fabrication by additive manufacturing processes.

### BACKGROUND

Additive manufacturing processes involve the buildup of one or more materials to make or form an object. AM encompasses various manufacturing and prototyping techniques known under a variety of names, including freeform fabrication, 3D printing, rapid manufacturing, and rapid prototyping. AM machines are capable of fabricating components having complex geometries from a wide variety of materials. The fabricated components correspond to computer-aided design (CAD) models used to prepare machine code readable by the AM machines to produce the components. Certain types of AM processes, such as Selective laser sintering (SLS), direct metal laser sintering (DMLS), selective laser melting (SLM), and electron beam melting (EBM), use an energy beam, for example, an electron beam or electromagnetic radiation such as a laser beam, to sinter or melt sequential layers of a fine powder material, creating a solid three-dimensional object in which particles of the powder material are bonded together. Another AM process is magnetohydrodynamic (MHD) drop-on-demand ejection and liquid droplet deposition, such as the MagnetoJet printing process commercialized by Xerox Corporation, in which droplets formed from metal wire feedstock are deposited from a nozzle in a molten state and coalesce and solidify upon reaching a heated and moving substrate or formed layers to form a build structure.

Components fabricated by AM processes often require post-processing and in particular surface treatments to roughen, smoothen, harden, or otherwise treat the outer surfaces of these products. Commonly, AM products have slightly rough surfaces upon initial completion. These rough surfaces are generally removed by surface treatment techniques, such as polishing and drag finishing, which provide a consistently smooth finish across treated surfaces. Other surface treatments include plasma spraying which is often used to incorporate porous coatings such as for medical implants.

However, such treatments can be problematic for products particularly designed to have varying surface finishes. For example, hip stem implants commonly have smooth surfaces meant to reduce irritation to soft tissue and rough surfaces designed to stimulate bone growth between the implant and the bone in particular areas. To achieve varied surface features, surfaces of these types of articles must undergo different treatments during fabrication, particularly during post-processing. For example, post processing of these articles often requires the cumbersome steps of machining, masking, and exposing an area to be treated before a surface treatment is applied. Fabricated articles sometime require multiple surface treatments (e.g., polishing of one surface region and plasma spraying onto another surface region).

Thus, there is a need to more efficiently isolate surface treatment to desired regions of products fabricated by AM processes.

### BRIEF SUMMARY

In accordance with an aspect, a build structure with a varying surface roughness may be formed along with a protective shield by a process. The protective shield may include temporary braces that may attach the protective shield to the build structure. The temporary braces may have a breaking point to facilitate removal of the protective shield. The protective shield and the temporary braces may be formed along with the build structure. The protective shield may be formed over an isolated region of the build structure and thereby may insulate that region from post processing treatments or other conditions. The temporary braces may be formed between the protective shield and the build structure to provisionally affix the protective shield to the build structure. Irrespective of their location, the temporary braces may be formed such that they provide an attachment between the protective shield and the build structure strong enough to endure post processing but fragile enough to be severed thereafter. The protective shield, while attached to the build structure, preserves the surface conditions of the covered region during post processing by providing a barrier between post processing mediums and the covered region. After post processing when the post processing involves polishing, the uncovered surface areas of the build structure may be smooth due to the polishing while the covered areas of the build structure maintain a rougher surface due to the protective shield. In this manner, implant structures designed with rough and smooth surfaces can be more efficiently fabricated by an AM process without adding additional steps to post processing. This process and the in-process structures fabricated thereby may be useful in the fabrication of medical implants, such as shoulder implants and patellofemoral implants, that utilize rough surfaces in certain areas to encourage bone growth and smooth surfaces in other areas to reduce skin irritation associated with the implant.

In another aspect, a build structure may be fabricated by a process. In the process, a first build structure portion of the build structure may be formed. Then a plurality of porous portion struts may be formed that define a second build structure portion of the build structure directly attached to the first build structure portion. Next, breakaway struts may be formed that directly attach to either one of or both of the first build structure portion or the second build structure portion. Each of the breakaway struts may have breakaway strut diameters less than porous portion strut diameters of the porous portion struts. Subsequently, a cover may be formed that may be attached to each of the breakaway struts such that the cover overlies the second build structure portion.

In some arrangements, the breakaway struts may be fractured to separate the cover from the build structure without fracturing either one of the first build structure portion or the second build structure portion.

In some arrangements, at least part of the first build structure portion of the build structure may be polished without any part of the second build structure portion being polished. In some arrangements, at least part of the second build structure portion may be polished. In some arrangements, at least part of the first build structure may be dragged, vibrated, chemically etched, tumbled, or machined. In some arrangements, at least part of the first build structure may be aggressively blasted. In some arrangements, at least part of the first build structure may be electropolished or electrochemically polished.

In some arrangements, a coating to at least part of the cover may be applied without the coating being applied to the second build structure portion. In some arrangements, at least part of the cover may be electroplated or thermal sprayed.

In some arrangements, the build structure is in the form of a medical implant. In some arrangements, the medical implant is in the form of a shoulder implant, a hip stem component, a patellofemoral component, a tibial component, a spinal implant, a knee implant, a trauma plate, a foot implant, an ankle implant, or an acetabular cup.

In some arrangements, the build structure may be fabricated by additive manufacturing.

In some arrangements, the first build structure portion may be solid, and the second build structure portion may be porous.

In some arrangements, when the cover is formed, a plurality of drain holes may be configured for powder removal.

In some arrangements, at least a portion of the build structure may be surface treated while the cover overlies the second build structure portion. In some arrangements, at least a portion of the build structure may be sprayed with plasma after the cover is removed. In some arrangements, at least a portion of the build structure may be surface treated with an arc deposition treatment after the cover is removed.

In accordance with another aspect, a build structure may be fabricated by a process. In the process, a first build structure portion of the build structure may be formed, and then a second build structure portion of the build structure may be formed. Next, breakaway struts attached to the build structure may be formed, and then a cover attached to the breakaway struts may be formed such that the cover overlies the second build structure portion. Subsequently, the build structure may be surface treated while the cover is attached to the build structure, and then the cover from the build structure may be separated after the surface treating step.

In some arrangements, a plurality of holes may be formed in the cover that are smaller than a medium used in the surface treating step.

In some arrangements, the build structure may be heat treated before the polishing step.

In some arrangements, the build structure may be machined before being separated from the cover.

In some arrangements, when the cover is formed, a plurality of holes may be formed in the cover. In some arrangements, powder may be removed through the plurality of holes in the cover.

In accordance with another aspect, an in-process build structure may include a first build structure portion, a third build structure portion, and a first cover. The third build structure portion may be attached to the first build structure portion and may be defined by a plurality of porous portion struts. The first cover may at least partially overlie the third build structure portion and may be attached to a remainder of the build structure by breakaway struts that may have breakaway strut diameters less than porous portion strut diameters of the porous portion struts.

In some arrangements, the build structure may be polished.

In some arrangements, the build structure may be in the form of a medical implant. In some arrangements, the medical implant may be in the form of a shoulder implant, a hip stem component, a patellofemoral component, a tibial component, a spinal implant, a knee implant, a trauma plate, a foot implant, an ankle implant, or an acetabular cup.

In some arrangements, the build structure may further include a fourth build structure portion and a second cover overlying the fourth build structure portion.

In some arrangements, the struts of the cover may be attached to a lower edge of the first build structure portion.

In some arrangements, the cover may include a plurality of holes configured for powder removal.

In some arrangements, the surface roughness of the first build structure portion and the third build structure portion may be different. In some arrangements, the first build structure portion may be a solid portion and the third build structure portion may be a porous portion.

### BRIEF DESCRIPTION OF THE DRAWINGS

A more complete appreciation of the subject matter of the present disclosure and the various advantages thereof may be realized by reference to the following detailed description which refers to the accompanying drawings, in which:
FIG. 1 is a perspective view of a build structure with a cover, in accordance with an aspect;
FIG. 2 is a perspective view of the build structure of FIG. 1 following removal of the cover, in accordance with another aspect;
FIG. 3 is a cross-sectional view of the build structure along lines 1-1 on the cover of FIG. 1;
FIG. 4 is an enlarged view of the layers of the build structure within the region of FIG. 3 shown in broken lines;
FIG. 5 is a side view of breakaway struts, in accordance with another aspect;
FIG. 6 shows perspective views of physical versions of the build structure of FIG. 2 fabricated by additive manufacturing;
FIG. 7A is a perspective view of a build structure with a cover, in accordance with another aspect;
FIG. 7B is a cross-sectional view of the build structure of FIG. 7A;
FIG. 7C is an enlarged view showing the layers of the build structure within the region of FIG. 7B outlined with broken lines;
FIG. 7D is another cross-sectional view of the build structure along lines 7D-7D of FIG.7A;
FIG. 8 is a perspective view of the build structure of FIG. 7A following removal of the cover, in accordance with another aspect;
FIG. 9 is a perspective view of a build structure with a cover, in accordance with another aspect;
FIG. 10 is a perspective view of the build structure of FIG. 9 following removal of the cover, in accordance with another aspect;
FIG. 11 is a process flow diagram of a process for fabricating a build structure with a solid portion, a porous portion, and a cover in accordance with another aspect; and
FIG. 12 is a process flow diagram of a process for fabricating a build structure with a varying surface treatment in accordance with another aspect.

### DETAILED DESCRIPTION

This disclosure relates to producing a build structure having a surface with a variable finish. The various embodiments described herein refer to in-process build structures with isolated surfaces and methods for obtaining a variable surface finish on build structures that are produced through additive manufacturing (AM) such as electron-beam additive manufacturing (EBM) machine or a laser melting machine, e.g., a direct metal laser melting machine, that may require post processing that may include a finishing treatment, e.g., drag finishing, rotary bowl, aggressive blasting, etc., and surface treatment, e.g., heat treatment, chemical polishing, physical polishing, electropolishing, or any combination thereof. Additionally, post processing may include various deposition treatments, e.g., arc deposition, chemical vapor deposition, physical vapor deposition, for depositing coatings on build structures. When applied to isolated regions of a build structure, such post processing techniques create a differing surface finish. For example, a build structure with a variable finish may include a solid portion, a porous portion, and a cover.

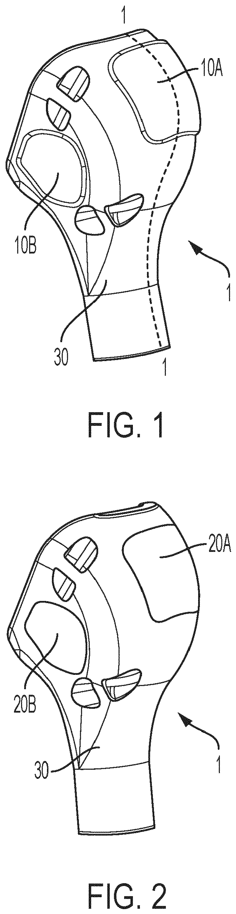

Referring now to the drawings, as shown in FIGS. 1-4, build structure 1 generally includes covers 10A and 10B, porous portions 20A and 20B, solid portion 30, and breakaway struts 11. Covers 10A and 10B overlie porous portions 20A and 20B and are each attached to solid portion 30 or respective ones of porous portions 20A and 20B. Porous portions 20A and 20B may be defined by porous portion struts (not shown), such as porous structures formed by struts in the form of those described in U.S. Patent Nos. 11,510,783 and 10,716,673, the disclosures of which are hereby incorporated herein by reference. In the example shown, the portion porous struts of porous portions 20A and 20B attach to solid portion 30 such that the porous portions are affixed to solid portion 30 around respective perimeters of the portion portions, although in some other arrangements, porous portions may be open on one or more sides of the porous portions such that struts of the porous portions have free ends (not shown) not attached to the solid portion (e.g., to create a barb effect). As in the example shown, solid portion 30 extends throughout an entirety of build structure 1 except for the regions through which porous portions 20A and 20B extend such that the combination of the solid portion and the porous portions forms an entirety of the build structure. Solid portion 30 may have other features adjacent to or extending through porous portions 20A and 20B and covers 10A and 10B, as described in more detail below.

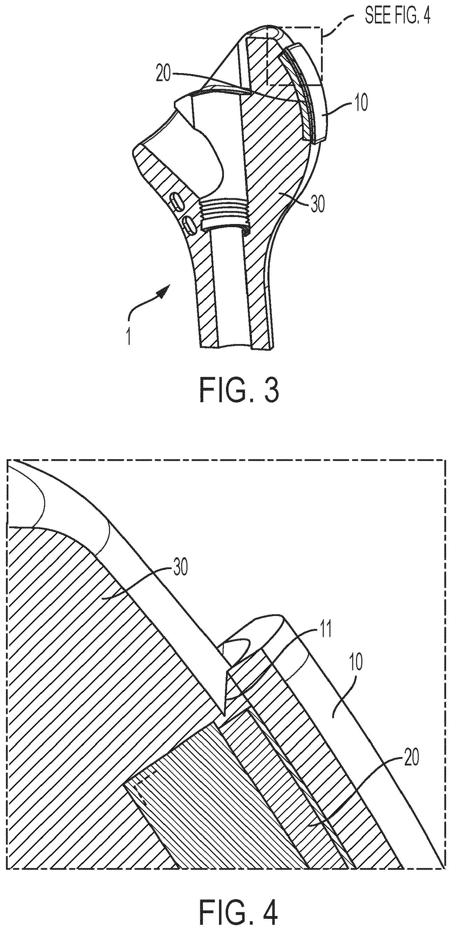

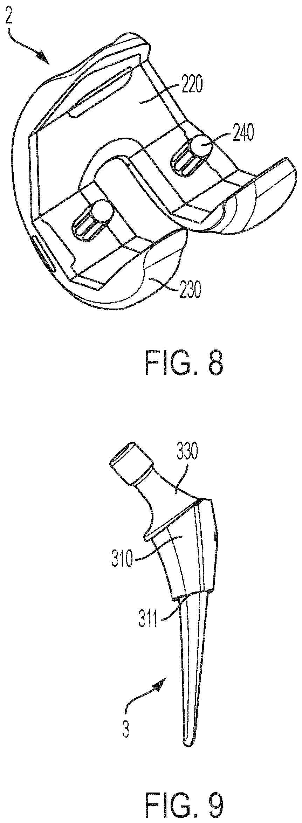

With specific reference to FIGS. 3 and 4, cover 10 extends over porous portion 20 such that the margins of the cover overlie a portion of solid portion 30. An outer surface of porous portion 20 is level with an outer surface of solid portion 30. In this manner, cover 10 forms an outermost surface of an in-process build structure that includes build structure 1. Cover 10 may be attached to solid portion 30 by breakaway struts 11. In the example shown, breakaway struts 11 are formed between solid portion 30 and cover 10 at the margins of cover 10 such that breakaway struts 11 connect to cover 10 and to solid portion 30. In some alternative arrangements, breakaway struts may be formed to extend from a porous portion to a cover, as described for example with respect to FIG. 9.

In some alternative arrangements, a cover may protect only a select region of a porous portion. In such instances, at least part of the perimeter of the cover may be supported by breakaway struts connected to the porous portion and to a part of the margin of the cover that extends over the porous portion.

In the example of FIGS. 1-4, cover 10 is a solid structure, although in some arrangements the cover may be partially or entirely porous. When the cover is a porous structure, the areas or shapes defined by the pores of the cover may be configured to isolate porous portion 20 from a medium or media, e.g., polishing tools or an abrasive solution, used for surface treatment of solid portion 30. In some such arrangements, the porous structure of the cover may be a non-permeable protective layer preventing any flow therethrough. In other instances, the cover may have a porous structure that is permeable to powders and fluids. In this manner, powder, such as a metal powder, used in the fabrication of a build structure and remaining in the porous portion may be drained out from the porous portion through the pores of the cover.

Whether breakaway struts 11 are attached to a porous portion such as porous portion 20 or a solid portion such as solid portion 30, the breakaway struts and a set of such breakaway struts may come in various forms and may be broken away along with a cover such as cover 10 so as to leave only a build structure such as build structure 1 so long as the breakaway struts are formed to break upon sufficient pressure applied to either or both the cover or the breakaway struts themselves where such sufficient pressure does not break or otherwise fracture the one or both of the solid and porous portions to which the cover is attached by the breakaway struts. In some arrangements, breakaway struts 11 may have a diameter along a length of the struts or at least a connecting end of the breakaway struts attached to either a porous portion or a solid portion that is less than a diameter of the porous portion struts. In some such arrangements, breakaway struts 11 may have a uniform diameter, which may be the same diameter for all of the porous portion struts of porous portion 20. In some arrangements, breakaway struts 11 may have an aspect ratio, i.e., a ratio of width/diameter to length, that is less than an aspect ratio of the porous portion struts. In some arrangements, breakaway struts 11 may be made of a different material, such as one providing a lower bending strength, than the porous portion struts. In some arrangements, breakaway struts may be fabricated in an additive manufacturing process at either one or both a different temperature setting or different dwell time than the porous portion struts are fabricated such that metal powder particles forming the breakaway struts have a weaker bond to each other than metal powder particles forming the porous portion struts. Of course, any combination of these differences between breakaway struts 11 and the porous portion struts is contemplated in accordance with the present disclosure. Through any one or any combination of these arrangements, each of breakaway struts 11 may be broken, such as by a sufficient bending force, without fracturing either porous portion 20 or solid portion 30 to which the respective struts are attached to facilitate a temporary attachment between cover 10 and the solid portion 30. In contrast, a configuration of porous portion struts of porous portion 20 is sufficient to provide a permanent support and attachment to solid portion 30 of porous portion 20. As such, the porous portion struts will not break and porous portion 20 will not detach from solid portion 30 when breakaway struts 11 are detached (i.e., broken).

In some arrangements, breakaway struts 11 may overlap one another when formed such that a continuous wall is formed by the breakaway struts. In such instances, the continuous wall provides a protective barrier along with cover 10 and an attachment between cover 10 and build structure 1. Alternatively, breakaway struts 11 may be spaced apart such that they form individual fiber-like structures attaching to and supporting regions, such as the margins, of cover 10 to build structure 1. In such instances, the gaps between breakaway struts 11 may be smaller than mediums, e.g., ceramic media, sand media, glass, corn granules, etc., used during post processing. In this manner, individual breakaway struts 11 still provide a protective barrier between porous portion 20 and post processing mediums despite the gaps between breakaway struts 11.

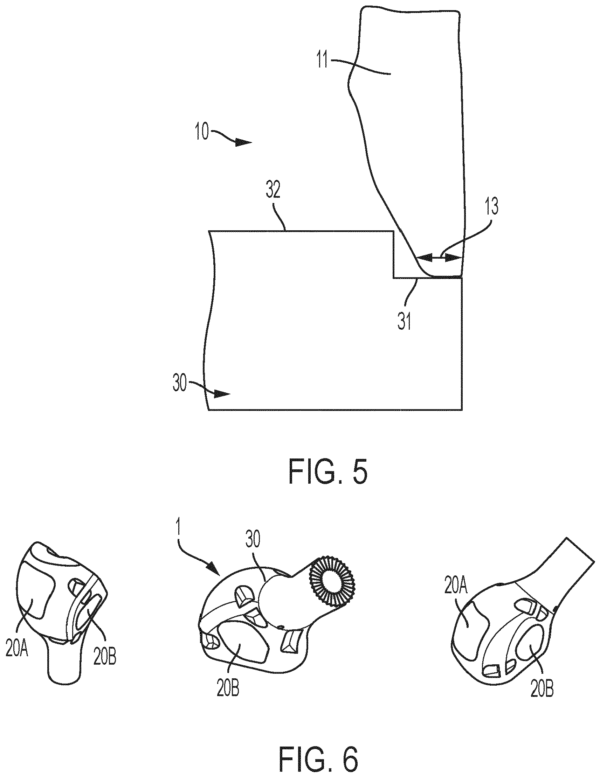

Now referring to FIG. 5, solid portion 30 may be formed to have a notch defining an attachment ledge 31 providing an attachment location for breakaway struts 11 of cover 10. In such instances, at least some of breakaway struts 11 may be formed on and connected to attachment ledge 31. Attachment ledge 31 may be configured such that it sits lower than outer surface 32, and vice versa. When cover 10 is removed by breaking breakaway struts 11, the breakaway struts may leave behind remnants of the strut structure. In some arrangements, attachment ledge 31 may be set at a depth below outer surface 32 such that the remnants of the strut left behind are below outer surface 32. To control breakage of breakaway struts 11, the width or diameter of breakaway struts 11 may be configured to facilitate easy and predictable breakage of breakaway struts 11 and to prevent damage to porous portion 20 and solid portion 30 during removal of cover 10. For example, breakaway struts 11 may have a varying width or diameter with narrowed region 13 that provides a breaking point for breakaway struts 11. Narrowed region 13 (i.e., the breaking region) may be located at any point along the length of breakaway strut 11, including at the interface of the strut and solid portion 30, as shown in FIG. 5. Narrowed region 13 is a section that has less rigidity compared to the rest of the strut due to narrowed region 13 having less structural material and thereby provides a breaking point for breakaway struts 11. In this manner, narrowed region 13 may be used to control where breakaway struts 11 break and how much of the structure of each breakaway strut is left attached to the solid portion 30 or porous portion 20 to which the respective breakaway strut is attached following the detachment of cover 10. Additionally, breakaway struts 11 may have an expanded section (e.g., as shown on the top of breakaway strut 11 in FIG. 5) adjacent to cover 10 that resists bending and thereby helps to maintain an attachment of the expanded section with cover 10 during removal of the cover. Tapering of breakaway struts 11 allows them to pass by outer surface 32 and attach to ledge 31 without interfering with solid portion 30, as illustrated by the strut shown in FIG. 5. It is contemplated that breakaway struts 11 may be formed in a variety of shapes and sizes with enough rigidity to withstand post processing.

In arrangements including attachment ledge 31, porous portion 20 (not shown in FIG. 5) may be level with attachment ledge 31 or with outer surface 32 of solid portion 30. In either instance, breakaway struts 11 may be formed such that they have no connection or contact with the porous portion. Alternatively, breakaway struts 11 may be formed such that they adjoin both solid portion 30 and porous portion 20 of the build structure. In this manner, breakaway struts may be placed wherever necessary to provide optimal support for cover 10.

Now referring to FIG. 6, build structure 1 can be formed (e.g., printed) from metallic materials. Covers 10A, 10B, porous portions 20A, 20B, and solid portion 30 may all be formed out of the same material or have their own physical composition made from, for example, but no limited to, titanium, titanium alloys, stainless steel, etc.

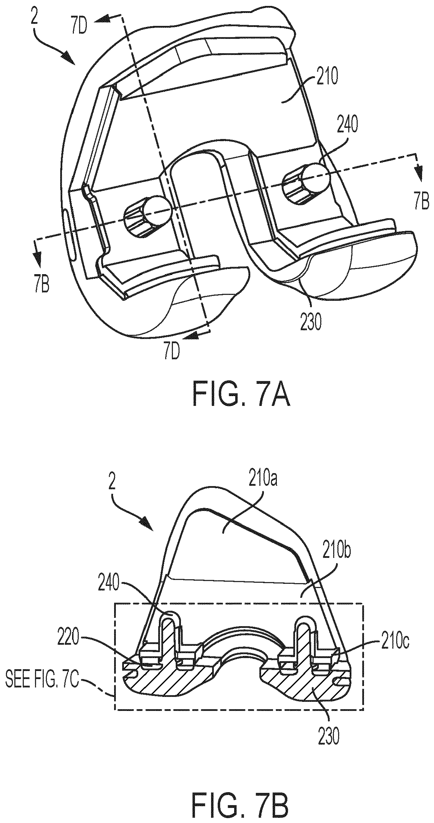

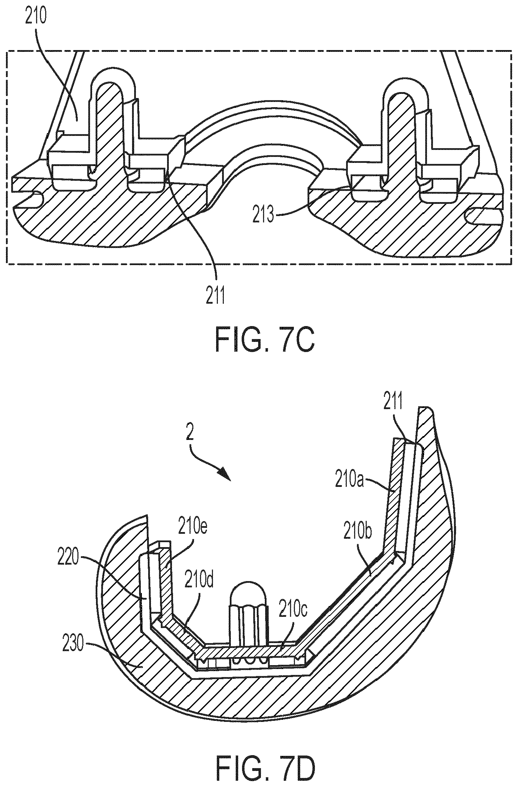

Referring now to FIGS. 7A and 8, in some arrangements, a solid portion may have features that extend through both the cover and porous portion when the cover is covering the porous portion. In the example shown, an in-process knee implant includes elongated feature 240 that extends from solid portion 230 and through both porous portion 220 and cover 210. In such instances, cover 210 may adjoin via breakaway struts with elongated feature 240 and breakaway struts 11 arranged such that tools or other mediums used during post processing do not protrude into the region between cover 210 and elongated feature 240. In this manner, additional features extending from the solid portion may overlap with or extend through covered porous portions without impacting the efficacy of the protective barrier of the cover.

Now referring to FIGS. 7A-7D, in some arrangements, a cover may be supported by segments connected to a porous portion, and multiple covers may be used to cover a porous portion. In this example, covers 210a-210e are supported by segments 211. Segments 211 are solid support structures extending along the edges of covers 210a-210e. Segments 211 attach to covers 210a-210e on one side of the segments. Segments 211 taper to narrow region 213 which are attached on another side of the segments to porous portion 220 adjacent to solid portion 230. The solid structure and narrow region 213 of segments 211 facilitate a clean break when covers 210a-210e are removed from build structure 2. To prevent post processing agents from reaching porous portion 220 at bends of build structure 2, covers 210a-210e overlap adjoining covers such that no gaps are formed therebetween. For example, as shown in FIG. 7D, cover 210a overlaps cover 21 0b, and cover 210b overlaps both cover 210a and cover 210c, and likewise with respect to the other covers. In this manner, multiple covers may be used to protect a porous portion from post-processing when the build structure includes chamfers or otherwise bends or curves, as shown in FIGS. 7A-8.

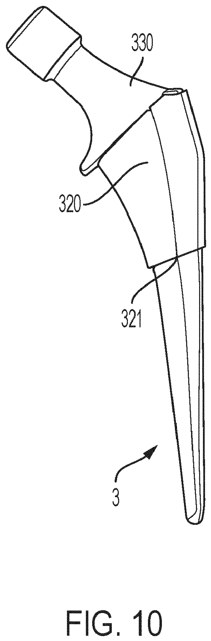

Now referring to FIGS. 9 and 10, in some arrangements, the outer surface of the porous portion may not be level with the outer surface solid portion 330. In this example, the outer surface of porous portion 320 extends above the outer surface of solid portion 330 (as shown in FIG. 10). As such, breakaway struts 311 are formed between cover 310 and porous portion 320 to attach and support cover 310. As cover 310 wraps around the porous portion 320, breakaway struts 311 are formed around the porous portion between the cover and the porous portion.

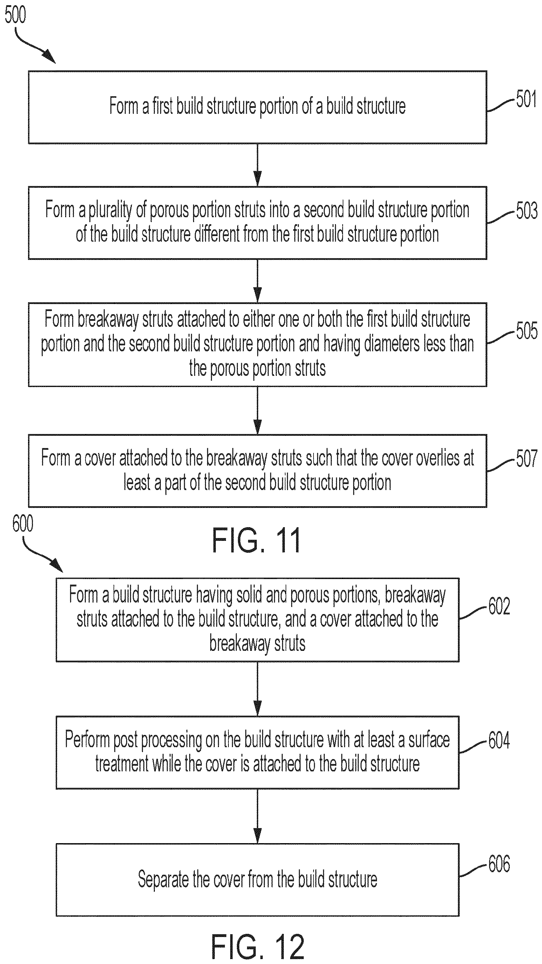

In reference to FIG. 11, a build structure is fabricated by process 500 to have a solid portion and a porous portion and to have a cover formed to overlie the porous portion. At step 501 of process 500, a first build structure portion of a build structure (e.g., solid portion 30 and build structure 1) is formed. At step 503, a plurality of porous portion struts are formed into a second build structure portion of the build structure different from the first build structure portion. At step, 505, breakaway struts (e.g., breakaway struts 11) are formed and attached to either one or both the first build structure portion and the second build structure portion, the breakaway struts having diameters less than the porous portion struts. At step 507, a cover (e.g., cover 10) is formed and attached to breakaway struts 11 such that the cover overlies at least a part of the porous portion. The cover creates a protective barrier that isolates the covered part of the porous portion from post processing media that may be used. In this manner, the covered part of the porous portion will have a different surface treatment, e.g., a rougher finish, compared to the other surfaces of the build structure when the build structure is exposed to post processing.

In reference to FIG. 12, a build structure is fabricated with a varying surface finish by process 600. At step 602 of process 600, a build structure having solid and porous portions, breakaway struts attached to the build structure, and a cover covering at least part of the porous portion and attached to the breakaway struts (e.g., build structure 1, solid portion 30, porous portion 20, breakaway struts 11, and cover 10, respectively) are fabricated, e.g., by additive manufacturing using an AM machine. At step 604, the build structure undergoes post processing which may include any one or any combination of a finishing process (e.g., drag finishing or blasting) and a surface treatment (e.g., polishing or heat treatment or machining). At step 606, the cover of the build structure is detached from the build structure (e.g., by breaking breakaway struts 11) such that no damage is done to any other part of the build structure. It is contemplated that the cover and porous portion may be fabricated by AM techniques as additions to prefabricated build structures. For example, the cover and/or the porous portion may be printed by AM techniques onto a solid portion prefabricated by casting, subtractive manufacturing, or other known manufacturing processes. In this manner, mass produced parts can be later modified with porous portions and covers that facilitate fabricating custom implant structures with varying surface finishes.

It is to be understood that the disclosure set forth herein includes any possible combinations of the particular features set forth above, whether specifically disclosed herein or not. For example, where a particular feature is disclosed in the context of a particular aspect, arrangement, configuration, or arrangement, that feature can also be used, to the extent possible, in combination with and/or in the context of other particular aspects, arrangements, configurations, and arrangements of the technology, and in the technology generally.
Furthermore, although the technology herein has been described with reference to particular features, it is to be understood that these features are merely illustrative of the principles and applications of the present technology. It is therefore to be understood that numerous modifications, including changes in the sizes of the various features described herein, may be made to the illustrative arrangements and that other arrangements may be devised without departing from the spirit and scope of the present technology. In this regard, the present technology encompasses numerous additional features in addition to those specific features set forth in the claims below. Moreover, the foregoing disclosure should be taken by way of illustration rather than by way of limitation as the present technology is defined by the claims set forth below.

## Claims

1. A method for fabricating a build structure, comprising the steps of:
forming a first build structure portion of the build structure;
forming a plurality of porous portion struts to define a second build structure portion of the build structure directly attached to the first build structure portion;
forming breakaway struts directly attached to either one of or both of the first build structure portion or the second build structure portion, each of the breakaway struts having breakaway strut diameters less than porous portion strut diameters of the porous portion struts; and
forming a cover attached to each of the breakaway struts such that the cover overlies the second build structure portion.

2. The method of claim 1, further comprising the step of:
fracturing the breakaway struts to separate the cover from the build structure without fracturing either one of the first build structure portion or the second build structure portion.

3. The method of claim 1 or claim 2, further comprising the step of:
polishing at least part of the first build structure portion of the build structure without polishing any part of the second build structure portion.

4. The method of claim 3, wherein the polishing step polishes at least part of the second build structure portion.

5. The method of claim 3 and claim 4, wherein the polishing step includes drag finishing, vibratory finishing, chemical finishing, tumble finishing, or machining.

6. The method of claims 3, 4, and 5, wherein the polishing step includes aggressive blasting.

7. The method of claims 3, 4, 5, and 6, wherein the polishing step includes electropolishing or electrochemical polishing.

8. The method of claims 1, 2, 3, or 4, further comprising the step of:
applying a coating to at least a part of the cover without applying the coating to the second build structure portion.

9. The method of claim 8, wherein the coating applying step includes electroplating or thermal spraying.

10. The method of claim 1, wherein the build structure is in the form of a medical implant.

11. The method of claim 10, wherein the medical implant is in the form of a shoulder implant, a hip stem component, a patellofemoral component, a tibial component, a spinal implant, a knee implant, a bone plate, a foot implant, an ankle implant, or an acetabular cup.

12. The method of any one of claims 1-11, wherein the forming steps are performed by additive manufacturing.

13. The method of any one of claims 1-12, wherein the first build structure portion is solid and the second build structure portion is porous.

14. The method of any one of claims 1-12, wherein the cover forming step forms a plurality of drain holes configured for powder removal.

15. The method of any one of claims 1-14, further comprising:
surface treating at least a portion of the build structure while the cover overlies the second build structure portion.
